# EUROPEAN PATENT APPLICATION

(11) **EP 1 582 215 A1**
(43) Date of publication of application: **05.10.2005**
(21) Application number: 03811948.3
(22) Date of filing: 27.11.2003
(51) Int. Cl.: A61K 38/00, A61P 9/00, A61P 9/10, A61P 43/00

(54) **METHOD OF INHIBITING CELL DEATH, CELL DEATH INHIBITOR AND REMEDY FOR DISEASE CAUSED BY CELL DEATH CONTAINING THE CELL DEATH INHIBITOR**

(30) Priority: 28.11.2002 JP 2002346325; 20.11.2003 JP 2003391243
(71) Applicant: Japan Science and Technology Agency, Saitama 332-0012 (JP)
(72) Inventor: OKADA, Yasunobu, Okazaki-shi, Aichi 444-0877 (JP); MORISHIMA, Shigeru, Sakai-gun, Fukui 910-0337 (JP); NABEKURA, Takashi, Miyazaki-shi, Miyazaki 880-0929 (JP); MANABE, Ken-ichi, Okazaki-shi, Aichi 444-0878 (JP); MORI, Shin-ichiro, Okazaki-shi, Aichi 444-0851 (JP)
(74) Representative: Müller, Frithjof E.
(86) International application number: PCT/JP2003/015201
(87) International publication number: WO 2004/047851

(57) **Abstract**

A method according to the present invention for inhibiting cell death includes administrating an anion channel-forming peptide to cells under lactacidosis. The anion channel-forming peptide forms an anion channel on a cell membrane artificially. A cell death inhibitor according to the present invention contains the anion channel-forming peptide. Some of examples of the anion channel-forming peptide are a VacA protein derived from *Helicobacter pylori* and a glycine receptor channel variant peptide. The method and cell death inhibitor according to the present invention are effective especially for necrotic cell death that accompanies cell swelling.

## Description

### TECHNICAL FIELD

The present invention relates to a method for inhibiting cell death caused by pathologic phenomenon due to blood supply impairment such as ischemia, especially, necrotic cell death associated with cell swelling under lactacidosis, a cell death inhibitor for inhibiting such cell death, and a therapeutic drug for treating disease due to such cell death.

### BACKGROUND ART

It is known that acidosis associated with accumulation of lactic acid occurs often if cerebral ischemia is caused in disease such as cerebrovascular accident and the like. This is called "lactacidosis". It is known that glial cells and neurons under lactacidosis will die to ischemia if the ischemic environment is not alleviated. Many drugs for inhibiting such cell death have been studied and developed.

It is known that volumes of these cells persistently increase under lactacidosis. The cell death associated with cell swelling is called "necrotic cell death". The inventors of the present invention have reported that the persistent cell swelling under lactacidosis due to inhibition of the volume-regulatory anion channel (at least as to the neurons and glial cells). It has been known that the volume-regulatory anion channel plays an essential role in Regulatory Volume Decrease (RVD) that occurs after cell swelling caused especially by low osmotic pressure (low expansion) stimulus or the like. Because of this, it is expected that there is a possibility to inhibit the persistent cell swelling by dosing a material that can play, in lieu of the volume regulatory anion channel, that function of the volume regulatory anion channel which is inhibited under lactacidosis.

Besides necrotic cell death accompanied with the cell swelling, there is another type of cell death: apoptotic cell death, accompanied with cell shrinking. From a research group of the inventors of the present invention and other groups it has been reported that a method for inhibiting activity of an anion channel by using an anion channel blocker is effective to inhibit the apoptotic cell death. Patent Document 1 (Japanese Patent Application Publication No. 2002-3402 (published on January 9, 2002) is one of examples of the reports. Patent Document 1 reports that there is a possibility that the method may be effective for treating diseases due to cell death.

As described above, many researches and developments have been carried out on the methods and drugs for inhibiting apoptotic cell death. Meanwhile there found no report on a method and a drug for inhibiting the necrotic cell death. That is, there is no practical example of a drug for inhibiting the necrotic cell death, the drug based on a mechanism of administration a material that can play the function of the volume regulatory anion channel in lieu thereof under lactacidosis, thereby to form an artificial anion channel for allowing permeation of only anions.

One of the reasons for the lack of the practical example of such drug and method is that quite a small number of anion-selective ionophores (anion ionophores) are known, even though a large number of ionophores, which are cation-selective or allow permeation of both cation and anion are known: e.g. gramicidin, nystatin and the like. An ionophore is a material that artificially forms an ion channel or ion transporter. However, recently it has been reported that e.g. VacA protein derived from *Helicobacter pylori* and a peptide derived from glycine receptor channel variant have a function as the anion ionophore (for VacA protein derived from Helicobacter pylori, see Non-Patent Document 1 (Cover TL, Blaser MJ, "Purification and characterization of the vacuolating toxin from *Helicobacter pylori*", J. Biol. Chem., 267, 10570-10575, 1992); for the peptide derived from glycine receptor channel variant, see Non-Patent Document 2 (Wallace DP, Tomich JM, Iwamoto T, Henderson K, Grantham JJ, Sullivan LP, "A synthetic peptide derived from glycine-gated Cl- channel induces transepithelial Cl- and fluid secretion", Am. J. Physiol., 272, C1672-C1679, 1997) and Non-Patent Document 3 (Mitchell KE, Iwamoto T, Tomich J, Freeman LC, "A synthetic peptide based on a glycine-gated chloride channel induces a novel choloride conductance in isolated epithelial cells", Biochim. Biophys. Acta., 1466, 47-60, 2000).

An object of the present invention is to provide a method for inhibiting cell death, a cell death inhibitor, and a therapeutic drug for treating disease caused by cell death, the drug containing the cell death inhibitor, the method, inhibitor and the drug based on a mechanism to form an artificial anion channel by using the anion ionophore.

### DISCLOSURE OF INVENTION

Considering facts that glial cell swelling and subsequent necrotic cell death, which are often seen under lactacidosis in ischemia, are caused by malfunction of volume-regulatory anion channels, and that a VacA protein derived from *Helicobacter* *pylori* forms an anion channel artificially, the inventors of the present invention accomplished the present invention based on finding that administration of the VacA protein to cells under lactacidosis inhibits the necrotic cell death.

A method according to the present invention for inhibiting cell death includes administrating an anion channel-forming peptide to cells under lactacidosis. Moreover, a cell death inhibitor contains an anion channel-forming peptide.

Ion channels exist on a cell membrane are proteins penetrating through the cell membrane. Each ion channel is an importance pathway for transporting a specific ion through the cell membrane. The ion channels play an important role for uptake and exhaustion of materials, and for giving and receiving information. The anion channel is one type of the ion channels, and allows permeation of only anion selectively. Therefore, what is meant by the "anion channel-forming peptide" is an exogenous peptide that artificially forms the anion channel as described above. Moreover, the peptide encompasses peptides made of various numbers of amino acid residues, that is, ranging from oligopeptide to polypeptide. Thus, the peptide encompasses proteins.

In general, cells restore their original volume in a while after forced to swell in volume by hypotonic challenge or the like. This phenomenon is called RVD (Regulatory Volume Decrease). However, it is known that under lactacidosis, which often occurs in brain ischemia occurred in crisis of brain infarct, brain neurons and glial cells (gliacytes) are swelled in volume persistently and do not restore their original volumes, resulting in later cell death. That is, the cell death is caused because no RVD occur due to inhibition of volume-sensitive anion channel in lactacidosis.

In the method and cell death inhibitor according to the present invention, the anion channel-forming peptide works to form an anion channel on a cell membrane artificially, thereby to cause the RVD. With this, the persistent cell swelling can be inhibited. As described in Example later described, this inhibits cell death, improving cell viability. Among cell death, the method and cell death inhibitor according to the present invention can inhibit especially necrotic cell death that is associated with cell swelling under lactacidosis.

It has been reported in Non-Patent Document 4 (Proceedings in the 78th Annual Meeting of Physiological Society of Japan, page 254, 1PA76; published March 1, 2001) by the inventors of the present invention that the VacA administration gives anion channels to glial cell swelled by lactacidosis, and RVD occurs in the swelled glial cells as a result of the formation of the anion channels.

However, Non-Patent Document 4 does not describe that the formation of the anion channels and resultant RVD significantly reduce the necrotic cell death and the inhibition of cell death caused by them are significant in prevention and therapy of disease. That is, it is firstly disclosed in the present invention that, as descried in later-described Example, the administration of the anion channel-forming peptide (VacA protein, glycine receptor channel variant peptide) significantly reduces the cell death, thereby improving cell viability.

Specific examples of the anion channel-forming peptide are a VacA protein derived from *Helicobacter pylori*, and glycine receptor channel variant peptide. As described in Example, it was confirmed that these inhibit the persistent cell swelling under lactacidosis actually.

In a method according to the present invention, the cell death may be judged based on nuclear stainability by cell membrane impermeable fluorescent dye propidium iodine. Moreover, in the cell death inhibitor according to the present invention, inhibition of the necrotic cell death may be evaluated according to loss of nuclear stainability by cell membrane impermeable fluorescent dye propidium iodide (PI). This indicates that in the present invention the judgment of the cell death may be carried out based on the nuclear stainability by using cell membrane impermeable fluorescent dye propidium iodide (PI). This judgment method of the cell death is described later in Example.

Moreover, in the method according to the present invention the cell death may be associated with significant reduction in mitochondrial dehydrogenase activity. Further, in the cell death inhibitor according to the present invention the cell death may be associated with significant reduction in mitochondrial dehydrogenase activity. This indicates that in the present invention the judgment of the cell death may be carried out based on the mitochondrial dehydrogenase activity. This judgment method of cell death is described later in Example as well.

With the judgment methods of the cell death, it is possible to easily judge effects of the inhibition of the cell death in using the method according to the present invention and in using the cell death inhibitor according to the present invention.

The method and cell death inhibitor according to the present invention can inhibit (i) the cell swelling occurred under lactacidosis in ischemia and (ii) the subsequent cell death (especially the necrotic cell death). The ischemic cell death occurs resulting in various ischemic disease. By preventing the ischemic cell death, it is possible to prevent malfunction of brains and hearts, and consequent death. That is, the method and cell death inhibitor are effectively used for prevention and treatment of various disease that is due to cell death. Therefore, the present invention encompasses a drug containing the cell death inhibitor and being used for treating disease caused by cell death.

The cell inhibitor can effectively inhibit the cell death of various cells, especially, glial cells. Thus, it is preferable that the drug according to the present invention containing the cell death inhibitor be used for treating disease caused by glial cell death.

For a fuller understanding of the nature and advantages of the invention, reference should be made to the ensuing detailed description taken in conjunction with the accompanying drawings.

### BRIEF DESCRIPTION OF THE DRAWINGS

Figure 1 is a graph illustrating results of a test for investigating how cell volumes of C6 glial cells were affected by lactacidosis, acidosis, and administration of lactic acid.
Figure 2 is a graph illustrating results of a test for investigating effects of lactacidosis, acidosis, and administration of lactic acid on RVD in C6 glial cells.

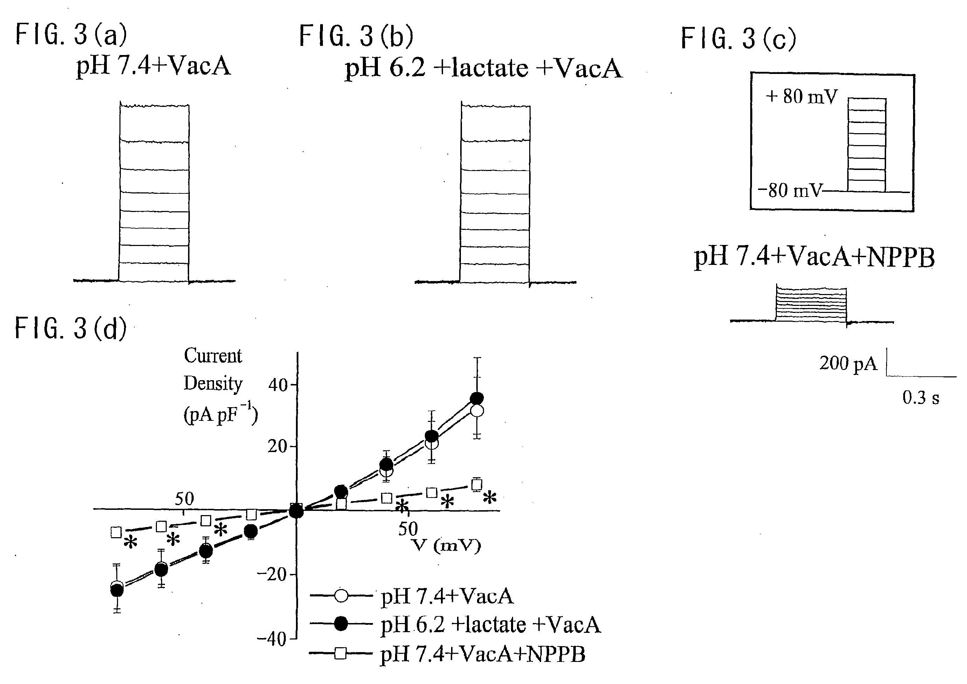

Figures 3(a) to 3(d) illustrate currents observed in C6 glial cells by whole-cell recordings, the C6 glial cell preincubated with administration of VacA. Figures 3(a) to 3(c) are graphs illustrating readings of currents in response to application of step pluses from -80mv to +80mV. Figure 3(a) illustrates readings with administration of VacA at pH 7.4 (control). Figure 3(b) illustrates readings with administration of VacA under lactacidosis. Figure 3(c) illustrates readings with administration of VacA and NPPB at pH 7.4. Figure 3(d) is a graph illustrating CURRENT-VOLTAGE relationship of instantaneous currents recorded under control (o), lactacidosis (•), and NPPB-containing condition (□).

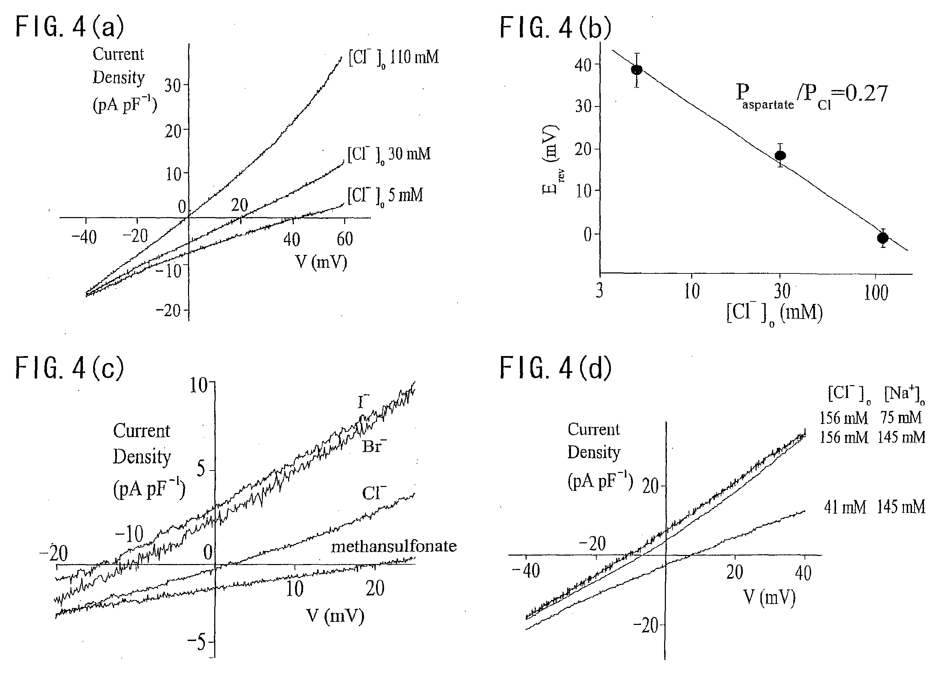

Figures 4(a) to 4(d) illustrate anion selectivity of VacA-induced currents observed in C6 glial cells by whole-cell recordings. Figure 4(a) is a graph illustrating CURRENT-VOLTAGE relationships measured by ramp pulse application. The CURRENT-VOLTAGE relationships respectively represent different extracellular Cl- concentrations. Figure 4(b) is a graph illustrating a relation between reversal potential and logarithms of the extracellular Cl- concentrations. Figure 4(c) is a graph illustrating effects of anion substitution on CURRENT-VOLTAGE curves. Figure 4(d) is a graph illustrating effects of cation and anion substitution on the CURRENT-VOLTAGE curves.
Figure 5 is a graph illustrating results of an experiment investigating how VacA or gramicidin administration changed cellular volume of C6 glial cells under lactacidosis-induced cell swelling.
Figure 6 is a graph illustrating results of an experiment investigating how administration of a glycine receptor channel variant peptide changed cellular volume of C6 glial cells under lactacidosis-induced cell swelling.
Figure 7 is a graph illustrating survival rates of C6 glial cells with or without the VacA administration under lactacidosis conditions.
Figure 8 is a graph illustrating survival rates of C6 glial cells under lactacidosis conditions (one hour treatment). Here, an activity of mitochondrial dehydrogenase was used as reference.
Figure 9 is a graph illustrating survival rates of C6 glial cells under lactacidosis conditions (two hour treatment). Here, an activity of mitochondria dehydrogenase was used as indicator.

### BEST MODE FOR CARRYING OUT THE INVENTION

In the following, the present invention is described in more details.

A method according to the present invention for inhibiting cell death is arranged such that an anion channel-forming peptide is administered to glial cells under lactacidosis. Moreover, a cell death inhibitor according to the present invention contains the anion channel forming peptide.

Examples of the anion channel-forming peptide are: VacA protein derived from *Helicobacter pylori,* a glycine receptor channel variant peptide, cholera hemolytic toxin, and other peptides.

The VacA protein is isolated from a toxin of *Helicobacter pylori* inhabiting in animal stomachs. It is known that the VacA protein form an anion channel, e.g., on artificial aliphatic bilayer membrane (c.f. Non-Patent Document 1). The VacA protein can be isolated according to a method described in Non-Patent Document 1.) The VacA protein encompasses not only a protein of a primary structure described in Non-Patent Document 1, but also proteins to/from which one or more amino acids are replaced, deleted, inserted, and/or added provided that the function to form the anion channel is not lost. That is, variant proteins of the VacA protein are encompassed in the anion channel-forming peptide.

Moreover, as described in Non-Patent Documents 2 and 3, the glycine receptor channel variant peptide is a polypeptide made of 23 amino acids of a second transmembrane region (M2) corresponding to pore formation portion of a glycine receptor a subunit, and having high water solubility by addition of four lysines to a carboxyl terminal. It has been reported that the glycine receptor channel variant peptide increases secretion of Cl- from an epithelial cell monolayer and increases of Cl⁻ conductance of epithelial cells. The glycine receptor channel variant peptide can be prepared and obtained according to method described in Non-Patent Documents 2 and 3.

Properties and preparation method of cholera hemolytic toxin are described in the following reference documents:
Reference Document 1: Zitzer A, Palmer M, Weller U, Wassenaar T, Biermann C, Tranum-Jensen J, Bhakdi S, ┌Mode of primary binding to target membranes and pore formation induced by Vibrio cholerae cytolysin (hemolysin)┘, Eur. J. Biochem., 247, 209-216, 1997; and
Reference Document 2: Moschioni M, Tombola F, de Bernard M, Coelho A, Zitzer A, Zoratti M, Montecucco C, ┌The Vibrio cholerae hemolysin anion channel is required for cell vacuolation and death┘, Cell. Microbiol., 4 (7), 397-409, 2002.

A cell death inhibitor according to the present invention can be used for prophylaxis or treatment for disease caused by cell death (especially preferably disease to which necrosis progress) for mammalians (e.g. human, mouse, rat, rabbit, dog, cat, cow, horse, pig, monkey, and the like). Examples of the disease are: ischemic disease such as cardiac infarction, cerebral ischemia, and the like; neurotic degenerative disease such as Alzheimer disease; Parkinson disease, and the like; congestive cardiac insufficiencies; and the like. As described in Examples later, the cell death inhibitor containing anion channel-forming peptide such as the VacA protein inhibits swelling of glial cells under post-ischemic lactacidosis. It is preferable to use the cell death inhibitor for the purpose of prophylaxis or treatment especially for ischemic disease among the disease mentioned above. Moreover, the cell death inhibitor according to the present invention is applicable for cases where plural types of disease occur as well as cases where only one type of disease occurs.

The cell death inhibitor according to the present invention contains only the "anion channel-forming peptide". it may be administered directly. However, in general, the cell death inhibitor according to the present invention may contain a carrier that is pharmacologically allowable. The cell death inhibitor may be produced by a conventionally known production method.

Various organic or inorganic carrier materials, which can be generally used as pharmaceutical raw materials, may be used as the carrier pharmacologically allowable. These may be blended therein as (i) an excipient, lubricant, binder, disintegrant, and the like, each of which is for solid drugs, or (ii) a solvent, solubilizing agent, suspending agent, isotonic agent, buffer, soothing agent, and the like, each of which is for liquid drugs. Moreover, VacA protein content as "anion channel-forming peptide" content in the cell death inhibitor is 0.2µg/ml to 7.5µg/ml preferably, and 2.5µg/ml to 7.5µg/ml more preferably. It is necessary that the VacA protein be activated with an acidic solution (pH 2) immediately before use.

In terms of dosage form, the cell death inhibitor according to the present invention may be a drug for oral administration, e.g., tablet, capsule (encompassing soft capsule, microcapsule), powder, granule, syrup, and the other type. Besides the drug for oral administration, the cell death inhibitor according to the present invention may be a drug for non-oral administration, e.g., injection, suppository, pellet, drops, and the other type. These are low in toxicity and can be administered orally or non-orally.

### [Example]

In the following, the present invention is described more specifically, referring to Example, which is not to limit the present invention. The present Example confirmed that VacA protein and a glycine receptor channel variant peptide, each of which is an example of an anion channel-forming peptide contained in a cell inhibitor according to the present invention, can inhibit cell swelling under lactacidosis. The following explains a method and results of a confirming experiment.

### (1) Experiment Method

### [Cell Culturing Method]

A C6 glial cell line of rat astroglia origin (c.f. Benda et al, "Differentiated rat glial strain in tissue culture", Science 161, 370-371, 1968) was obtained from American Type Culture Collection and grown as monolayers in petri dishes or a culture flask at 37°C in humidified air with 5% CO₂ in Dulbeco's modified minimal essential medium (DMEM). The medium was supplemented with 25mM bicadrbonate, 10(%) fetal calf serum, 100 IU/ml penicillin G, and 50mg/ml streptomycin. Monolayer cultured cells were harvested by trypsinization in phosphate-buffered saline (PBS) containing 0.05% trypsin and 0.02% EDTA. The cells were centrifuged for one minute at 800 rpm, and a supernatant was removed. To inactivate trypsinase, the cells were suspended in DMEM containing fetal calf serum, and after a second centrifugation, a supernatant was removed. Subsequently, the cells were suspended in serum-free medium containing the following electrolytes (in mM):
Electrolytes: 125mM NaCl, 2.5mM KCl, 2.0mM CaCl₂, 1.0mM MgCl₂, 1.25mM NaH₂PO₄, 25mM NaHCO₃.

This cell suspension was maintained at 37°C and pH 7.4 by bubbling with 95% air and 5% CO₂ until use in experiments.

### [Cell Volume Measurements]

Cell volume was assessed according to a Coulter principle using a flow cytometer (EPICS Elite ESP, Coulter Co., Miami, FL). Cell viability was also assessed under a flow cytometry by a propidium iodide exclusion test. The cell suspension of 10ml was incubated at 37°C fro 1 min after adding 2 µl of PBS containing 10 mg/ml propidium iodide. Mean cell volume was measured at a given time by introducing 350 µl of the cell suspension containing 2 × 10⁴ cells into the flow cytometer. A baseline cell volume was obtained as mean of values measured before and after a 20-min incubation in control solution (pH 7.4). Thereafter, effects of acidosis (pH 6.2), lactacidosis (pH 6.2), or lactate (pH 7.4) were assessed with addition of (i) HCl, (ii) lactate, or (iii) lactate and Tris. After the cells were preincubated with control (pH 7.4), acidic (pH 6.2), or lactacidosis (pH 6.2 with lactate) solution (all isotonic), effects of a hypotonic challenge were also assessed by diluting the isotonic medium with a 30% volume of deionized distilled water.

Composition of control solution employed for cell volume measurements was as follows: 125mM NaCl, 2.5mM KCl, 2mM CaCl₂, 1.0 mM MgCl₂, 10mM glucose, 1.25mM NaH₂PO₄, and 25mM NaHCO₃ (pH 7.4, in 95% air/5% CO₂). To examine the effects of lactacidosis, the medium pH was titrated by adding isotonic lactic acid solution so that a final concentration of lactic acid was 25mM. To examine the effects of acidosis, the medium pH was titrated to 6.2 with HCl. To observe the effect of lactate alone, the pH of the medium containing 25 mM lactic acid was adjusted to 7.4 by adding an appropriate amount (7.5 mM) of Tris. Molar osmolality measured by a freezing-point depression osmometer (Vogel, Giessen, Germany) was, 300, 306, and 312 mosmol/kg-H₂O.

### [Electrophysiology]

Using wide-tipped electrodes (to 2 MΩ), observation according to whole-cell recording (patch-clamp methods) was performed at room temperature (23-25°C) as reported previously (Kubo M., Okada Y., "Volume-regulatory Cl- channel currents in cultured human epithelial cells", J. Physiol. 456, 351-371, 1992). Series resistance (< 5 MΩ) was compensated (to 60% to 70%) to minimize voltage errors. Time course of current change was monitored by applying (every 15 s) alternating step pulses (1-2 duration) from a holding potential of 0 to ±40 mV. To monitor voltage dependence of a whole-cell volume-sensitive current, step pulses (2-s duration) were applied from a prepotential of -60mV (2-s duration) to test potentials of -60 to 100 mV in 20mV increments after attaining steady-state activation of swelling-induced current. To monitor the profile of a VacA-induced current, step pulses (300-ms duration) were applied from a prepotential of -80mV (300-ms duration) to test potentials of -80 to 80mV in 20mV increments. Instantaneous currents were recorded 2.5 ms after application of step pulses. To monitor current-voltage (I-V) curves of volume-sensitive or VacA-induced currents, ramp command pulse were applied for 0.5 s from - 40 to 60 mV. Currents were recorded using an EPC-9 amplifier (HEKA, Lambrechet, Germany). Current signals were filtered at 1 kHz and digitized at 4 kHz. Pulse+PulseFit software (version 8.11; HEKA) was used for command pulse control, data acquisition, and analysis.

For most whole-cell patch-clamp experiments, the following solution (electrolytic bath) and pipette solutions were used:
Isotonic (320 mosmol/gk H₂O) bathing solution: 100mM CsCl, 5mM MgSO₄, 90 mannitol, and 10 HEPES (pH 7.4 titrated with CsOH).

Cell swelling was induced by hypotonic (270 mosmol/kg H₂O) solution in which mannitol was reduced to 40 mM. The pipette (intracellular) solution (290 mosmol/kg H₂O) contained 110 CsCl, 2mM MgSO₄, 2mM Na₂ATP, 1mM EGTA, 60mM mannitol, and 1mM HEPES (pH 7.2 with CsOH). In some experiments, 10mM 1,2-bis(2-aminophenoxy)ethane-N,N,N',N'-tetra-acetic acid (BAPTA) was added to the pipette solution in place of 1 mM EGTA. To assess anion permeation properties of the volume-sensitive Cl- channel or the VacA-induced channel, chloride in the isotonic bathing solution was replaced with equimolar concentration of iodine, bromide, aspartate, or methansulfonate. Permeability properties of VacA-induced channels were also assessed by using the following solutions:
Isotonic (315 mosmol/kg H₂O) bathing solution: 145mM NaCl, 5mM KCl, 1mM MgCl₂, 10mM mannitol, 2mM CaCl₂, and 10mM HEPES (pH 7.4 titrated with Tris);
Pipette (intracellular) solution (300 mosmol/kg H₂O) contained 30mM NaCl, 80mM Kel, 2mM MgCl₂, 70mM mannitol, 1mM EGTA, 2mM Na₂ATP, 10mM HEPES (pH 7.2).

Relative anion permeability was also assessed by replacing 115 mM extracellular Cl- with aspartate. Relative cation permeability was observed by replacing 145 mM extracellular Na⁺ with 75 mM Na⁺ and 70 mM N-methyl-D-glucamine (NMDG).

### (2) Results and Discussion

### [Volumetric Change of Glial Cells]

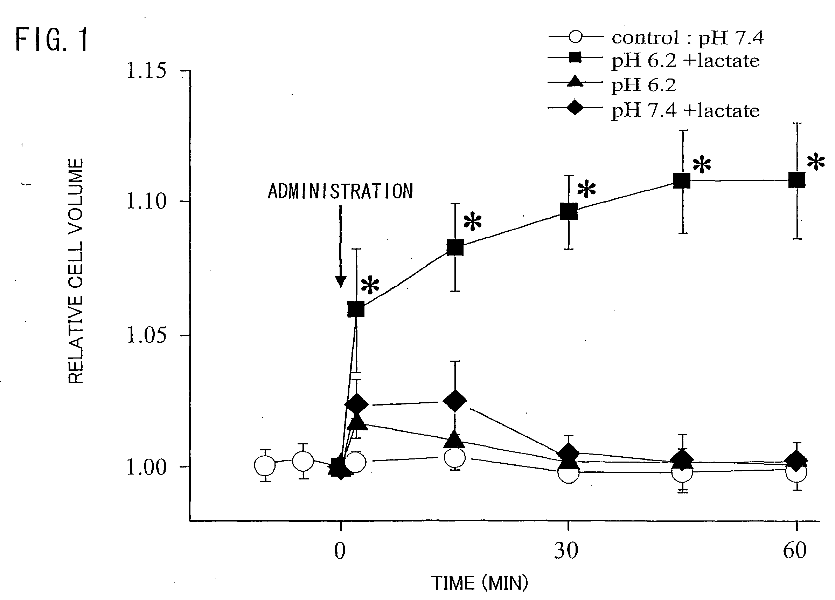

Figure 1 is a graph illustrating time course of cellular volume change of glial cells in relative cell volume (i) under control conditions of pH 7.4 (open circles), (ii) under lactacidosis (filled squares), (iii) under acidosis (filled triangles), and (iv) with administration of lactic acid (filled diamonds).

Under control conditions, the mean cell volume of C6 glial cells was 802.4 ±39.4µm³ and remained unchanged for 1 h, as indicated by "open circles" in Figure 1. Reduction of the medium pH to 6.2 without the addition of lactic acid (i.e. acidosis) induced little change in cell volume (Figure 1, "filled triangles"). In contrast, when the medium pH was decreased to 6.2 by addition of 25 mM lactic acid (i.e. lactacidosis), a significant increase in the cell volume was observed (Figure 1, "filled squares" ). Means cell volume immediately increased to 1.06±0.02 times that of the basal level after 2 min. After slow swelling, the means cell volume reached a plateau level within 45-60min (1.11±0.02 times after 60min). However, significant swelling was not induced by lactic acid alone, when the medium pH was maintained at 7.4 (Figure 1, filled diamonds). These results indicate that glial cell swelling is not induced by acidification alone nor by lactate alone, and lactacidosis induces the glial cell swelling.

### [Inhibition of RVD under lactacidosis]

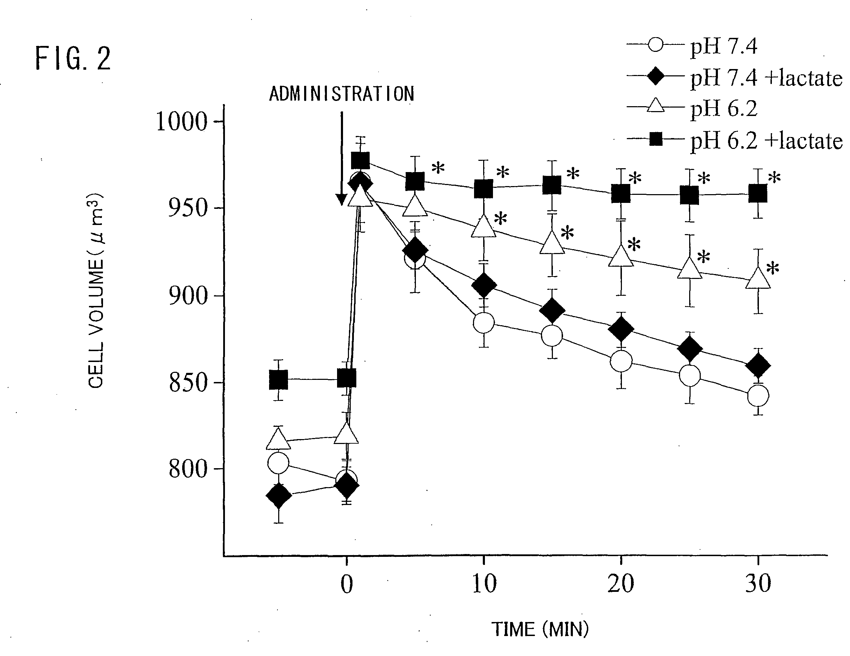

Figure 2 is a graph illustrating recordings of cell volumetric changes of C6 glial cells (i) under control conditions of pH 7.4 (open circles), (ii) with administration of only lactic acid (filled diamonds), (iii) under acidosis (open triangles), and (iv) under lactacidosis (filled squares),

When a hypotonic challenge (70% osmolality) was applied, C6 cells responded with transient swelling and subsequent volume recovery under control conditions (Figure 2, "open circles"). To examine whether the RVD was affected by lactacidosis, cells were preincubated with lactate-containing acidic (pH 6.2) isotonic solution and then exposed to the hypotonic solution while maintaining lactacidosis. As illustrated in Figure 2 (filled squares), the RVD was abolished under lactacidosis conditions. However, in the case where lactic acid was added at pH 7.4, the RVD occurred almost normally (Figure 2, filled diamonds). In the absence of lactic acid, the RVD was only partially inhibited by acidic conditions (pH 6.2, open triangles) .

As described above, it was found that the RVD, which occurs in normal cells after temporal cell swelling, did not take place under lactacidosis but the cells kept swelling persistently. This result indicates that the swelling under lactacidosis leads to necrotic cell death finally.

Moreover, even though no data is presented here, the present experiment confirmed that the persistent cell swelling is caused by inhibiting the function of volume regulatory Cl⁻ channel.

### [Anion Channel Formation on Glial Cell Membrane due to VacA Administration]

Explained below are results confirming VacA protein administration causes artificial formation of anion channels on glial cells. The VacA was purified from *Helicobacter pylori* 60190 according to the method described in Non-Patent Document 1, and pretreated with an acidic phosphate buffer (pH 2) at 37°C for 10 min before activation.

Incubation of C6 glial cells with 2.5 µg/ml VacA for 50 min at 37°C did not induce a change in cell morphology, as seen under a light microscope, presumably because no supplement weak bases (such as ammonium) were added to the medium, and did not affect cell viability, as judged by exclusion of propidium iodide from the cells (i.e. according to non-stainability). However, this resulted in activation of large current. The current did not exhibit time-dependent activation or inactivation kinetics (Figure 3(a)) and exhibited only slight outward rectification (Figure 3(d), "open circles"). The current profile (Figure 3(b)) and the I-V relation (Figure 3(d), filled circles) for VacA-induced currents recorded under lactacidosis conditions (pH 6.2) were indistinguishable from that for currents (Figure 3(d), "open circles") recorded under control conditions. VacA-induced currents were not affected by the inclusion of 10mM BAPTA in the pipette solution (data not shown). Extracellular application of NPPB (100 µM) strongly inhibited the VacA-mediated current (Figures 3(c) and 3(d), "□")

As described above, the administration of VacA to the glial cells formed the channel even under lactacidosis, and the current passing through the channel was observed by the patch clamp method (whole-cell method). Moreover, as illustrated in Figures 3(c) and 3(d), the current was inhibited by NPPB, which is known as a Cl- channel blocker. This proved that the current flow was due to the anion channel.

Explained below are results of an experiment that confirmed anion-selectivity of the VacA-induced current (i.e. there was an anion permeable channel) in C6 glial cells, as seen by the whole-cell method. Figure 4(a) is a graph illustrating current-voltage relation measured by ramp pulse application, for different extracellular Cl⁻ concentrations (5mM, 30mM, and 110mM). Figure 4(b) is a graph illustrating relation between reversal potential and logarithm of the extracellular Cl⁻ concentration. Figure 4(c) is a graph illustrating how the current-voltage curve was affected by replacing C^{l}- with I⁻, Br⁻, or methansulfonate-. Figure 4(d) is a graph illustrating how the current-voltage curve was affected if the cation and anion were replaced with other cation and anion respectively.

Reduction of [Cl⁻]₀ by replacement with aspartate induced rightward shifts in the reversal potential, indicating the anionic nature of VacA-mediated current (Figures 4(a) and 4(b)). Shifts in the reversal potential upon substitution of extracelullar Cl⁻ with other anionic species were also observed (Figure 4(c)). These results indicate anion permeability sequence of P_{I} > P_{Br} > P_{Cl} > P_{methansulfonate} > Pₐₛₚₐᵣₜₐₜₑ, as summarized in Table 1 below.

**TABLE 1**

| VacA-induced Anion Permeability of Anion Channels (Pₓ/P_{cl}) | |
|---|---|
| Anion (X-) | VacA |
| I- | 1.64±0.16 |
| Br⁻ | 1.13±0.14 |
| Cl⁻ | 1 |
| Methansulfonate⁻ | 0.45±0.14 |
| Aspartate⁻ | 0.27±0.08 |

Moreover, the VacA-induced channel was found to have only limited cation permeability (Pₙₐ/P_{cl} = 0.18±0.06), as only a slight shift in the reversal potential was observed upon the replacement of 70 mM extracellular Na⁺ with an equimolar concentration of NMDG (Figure 4(d)).

As described above, the present study showed that application of the *Helicobacter pylori* VacA increased the anion conductance of the C6 glial cell plasma membrane as illustrated in Figures 3 and 4. The VacA-induced channel permeability properties as presented in Table 1 are similar to those observed for VacA-induced channels in planar liquid bilayer membranes. In addition, in the present study, the VacA-induced anion current was found to be insensitive to lactacidosis (Figure 3) and independent of intracellular Ca²⁺. This result suggested that the VacA-related channel is also distinct from a Ca²⁺-activated Cl⁻ channel, which is expressed in a large number of cell types.

Summarizing these facts, it is concluded that VacA can form anion channels that are distinct from outward-rectifying and volume-serisitive anion channels in the plasma membrane of C6 glial cells and are functionally active even under lactacidosis conditions.

### [Recovery from Lactacidosis-Induced Cell Swelling with Pretreatment with VacA]

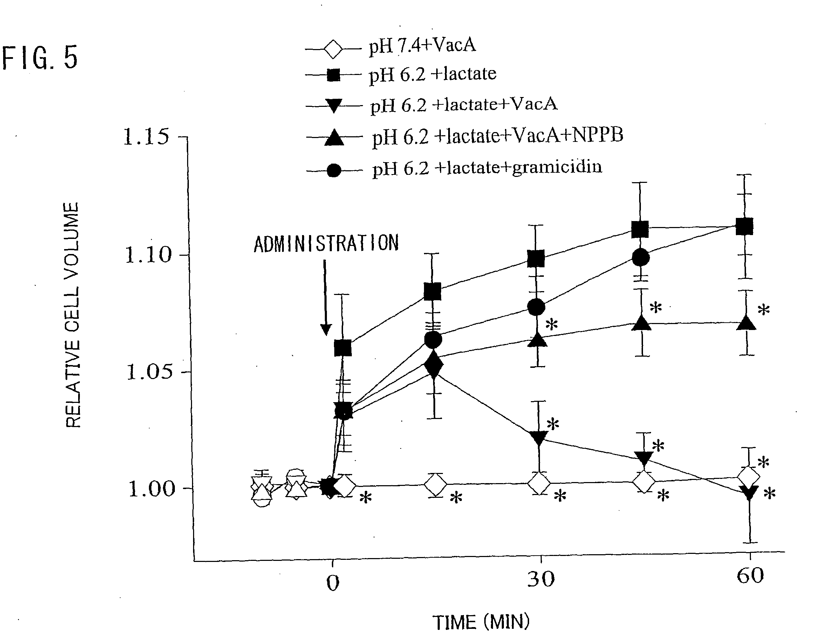

Next, explained referring to Figure 5 are experiment results on cell volumetric change in a case where the VacA or gramicidin (representing cation ionophore) was administered to the lactacidosis-cell swelling in C6 glial cell. Figure 5 illustrates a change in relative cell volumes against time (min) at the VacA or gramicidin administration. Suppose that a cell volume attained when the VacA was administered under normal conditions (without lactacidosis) is a basal level of the cell volume. The basal level is indicated by open diamonds in Figure 5.

Lactacidosis-induced cell swelling was less prominent in C6 glial cells pretreated with VacA (Figure 5, filled inverted triangles) in comparison with control cells (filled squares). VacA-treated cells responded to lactacidosis only with transient swelling to a peak of 1.05±0.02 times of the basal level after about 15 min. Subsequently, however, the cell volume gradually decreased in spite of continued lactacidosis, approaching to a level close to the basal level within 45-60 min (0.99±0.02 times after 60min). In the VacA-treated cells, the volume regulation after lactacidosis-induced transient swelling was abolished by 100 µM NPPB (Figure 5, filled triangles). In contrast, when C6 glial cells were pretreated with gramicidin (0.5 µM), lactacidosis-induced persistent cell swelling was not prevented (Figure 5, filled circles).

These results showed that an original cell volume was recovered by inhibition of the lactacidosis-induced persistent swelling by the preadministration of VacA, which forms an anion channel, but, the administration of gramicidin, which forms a cation channel did not inhibit the lactacidosis-induced persistent swelling and allowed persistent cell swelling. Moreover, the administration of NPPB, which is a channel blocker for inhibiting the volume-sensitive Cl⁻ channel, inhibited the function of the anion channel formed by the VacA administration and caused no cell volume reduction.

As described above, the present study proved that the occurrence of the VacA-induced anion selectivity confers the ability to regulate volume on C6 glial cells under continued lactacidosis conditions (Figure 5). These results indicate that a lactacidosis-induced impairment of the outward-rectifying and volume-sensitive anion channel is responsible for the RVD dysfunction under lactacidosis conditions.

### [Induction of RVD due to Administration of Glycine Receptor Channel Variant Peptide]

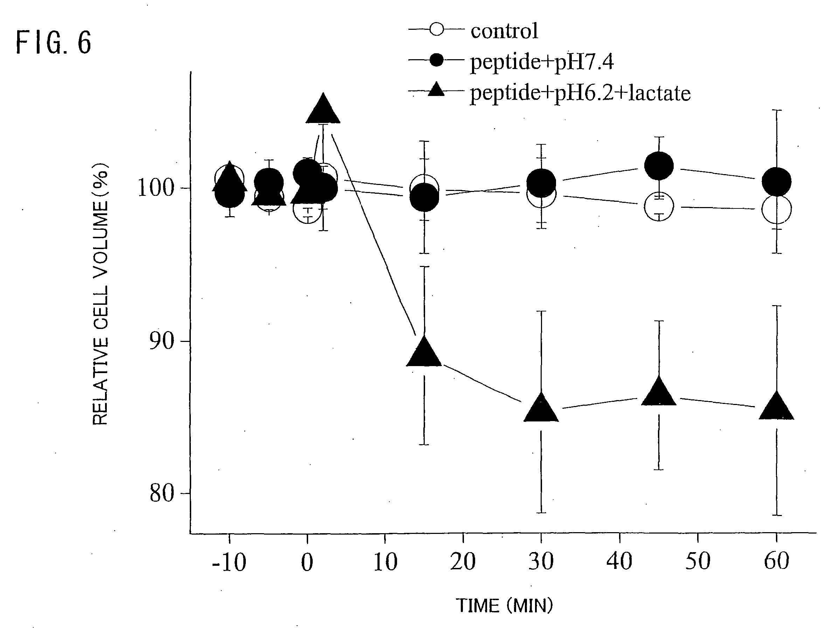

Next, explained below referring to Figure 6 are results of experiments on cell volumetric changes in case where the glycine receptor channel variant peptide, which is another anion channel-forming peptide was administered in C6 glial cells under lactacidosis.

In the graph of Figure 6, the cell volumetric change under control conditions (normal state, pH 7.4) is indicated by open circles. Cell volumetric change caused when the glycine receptor channel variant peptide was administered at pH 7.4 (normal state) is indicated by filled circles. Cell volumetric change caused when the glycine receptor channel variant peptide was administered under lactacidosis is indicated by filled triangles. As illustrated in Figure 6, it was found that the administration of the glycine receptor channel variant peptide to C6 glial cells inhibits persistent cell swelling under lactacidosis, and rather reduces the cell volume.

### [RVD-Induced Reduction in Cell Death under Cell Swelling]

Next, an experiment was performed to find whether administration of the VacA, which is an anion channel forming peptide, actually inhibited cell death in cells under lactacidosis. In the present Example, the cell death was measured by the following two methods.

### (a) Measurement of Cell Death based on Nuclear Stainability with Cell Membrane Impermeable Fluorescent Dye Propidium Iodine

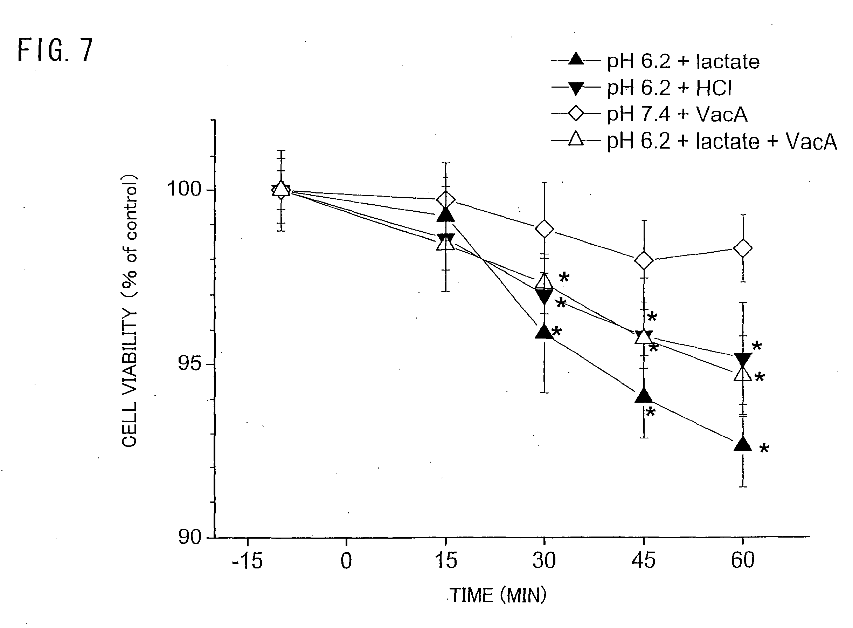

Firstly, effects of the VacA administration on necrotic cell death of C6 glial cells until one hour after lactacidosis was examined using, as an indicator, nuclear stainability with cell membrane impermeable fluorescent dye propidium iodide. Results are explained referring to Figure 7.

Figure 7 is a graph illustrating viabilities (%) of C6 glial cells under lactacidosis with or without the VacA administration, compared with control cells (under normal conditions at pH 7.4). The viabilities (here, a ratio of cells died due to necrosis and having PI stainability) were measured by using a flow cytometry. In Figure 7, viability of cells under lactacidosis (without the VacA administration ) is indicated by filled triangles. Viability of cells under acidosis is indicated by filled inverted triangles. Viability of cells (control) under normal conditions with the VacA administration is indicated by open diamonds. And viability of cells under lactacidosis with the VacA administration is indicated by open triangles. As illustrated in Figure 7, it was found that, under lactacidosis, cell viability was improved with the VacA administration (open triangles) than without the VacA administration (filled triangles). That is, it was found that viability tends to be improved even under lactacidosis, pretreatment with the VacA (50 min before) (the case of "open triangles").

Moreover, results of similar experiments with more cases are presented in Table 2.

**TABLE 2**

| Conditions | VacA Pretreatment | Viability at one hour after Lactacidosis | Significant Difference |
|---|---|---|---|
| Acidosis (pH6.2 HCl) | No | 95±0.8% (n=8) | - |
| Lactacidosis (pH6.2Lactic Acid) | No | 93±0.5% (n=8) | P< 0.001 |
| Lactacidosis (pH6.2 Lactic Acid) | Yes | 96±0.3% (n=8) | P>0.1 |

As seen in Table 2, the viability at one hour after lactacidosis was 93% without the VacA administration, but it was significantly improved to 96% with the VacA administration. There was no significant difference between the 96% viability under lactacidosis with the VacA administration and 95% viability under acidosis without lactic acid administration.

From these results, it was found that the administration of anion channel-forming peptide, such as the VacA or the like induces RVD in cells under lactacidosis and thereby the persistent cell swelling can be inhibited. Further, it was found that the inhibition of the persistent cell swelling inhibits cell death, thereby improving the cell viability. This proved efficacy of the method according to the present invention for inhibiting cell death by using the anion channel-forming peptide, and cell death inhibitor according to the present invention containing the anion channel-forming peptide.

### (b) Measurement of Cell Death based on Mitochondrial Dehydrogenase Activity

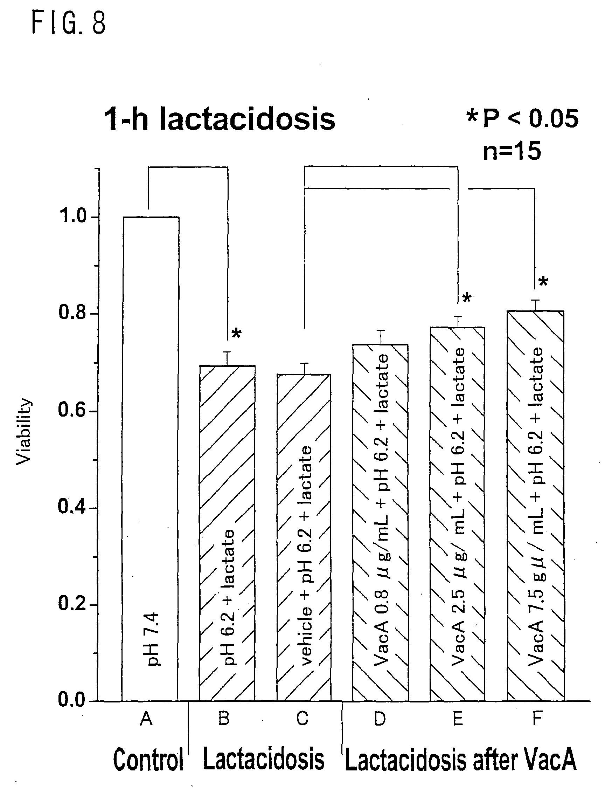

Next, it was examined that the inhibitory effects of exogenous anion channel VacA pretreatment on the cell death caused by lactacidosis (for one hour) could be reproduced in measurement using, as an indicator, mitochondrial dehydrogenase activity. Results are illustrated in Figure 8.

Figure 8 is a graph illustrating viability of C6 glial cells after exposing to lactacidosis conditions for one hour. In this graph, "A" is control (pH 7.4), "B" is lactacidosis (pH 6.2), "C" is lactacidosis (with solvent, pH 6.2), "D" is lactacidosis (pH 6.2) with the VacA administration (0.8 µg/ml), "E" is lactacidosis (pH 6.2) with the VacA administration (2.5 µg/ml), "F" is lactacidosis (pH 6.2) with the VacA administration (7.5µg/ml).

As illustrated in Figure 8, the dehydrogenase activity of C6 cells (i.e. cell viability) measured by MTT assay was reduced to approximately 70% after one-hour lactacidosis treatment (Figure 8, B and C). However, the reduction of the enzyme activity (viability) was alleviated to approximately 80% by the VacA pretreatment (Figure 8, E and F). This showed that the VacA pretreatment significantly inhibited the cell death

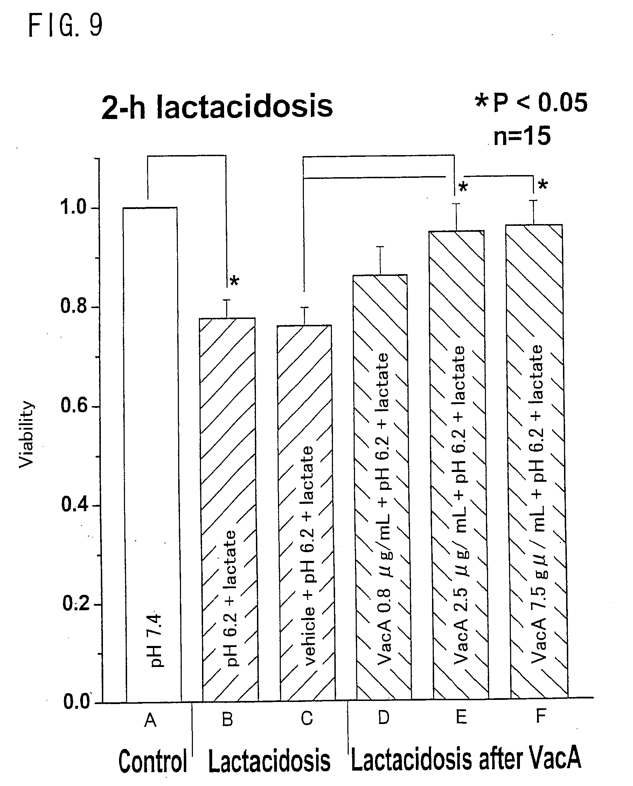

Moreover, it was also examined whether the VacA exhibits the inhibitory effect on glial cells under longer lactacidosis. Results are illustrated in Figure 9.

Figure 9 is a graph illustrating viability of C6 glial cells under two-hour lactacidosis. In this graph, "A" is control (pH 7.4), "B" is lactacidosis (pH 6.2), "C" is lactacidosis (with solvent, pH 6.2), "D" is lactacidosis (pH 6.2) with the VacA administration (0.8 µg/ml), "E" is lactacidosis (pH 6.2) with the VacA administration (2.5 µg/ml), "F" is lactacidosis (pH 6.2) with the VacA administration (7.5µg/ml).

As illustrated in Figure 9, similarly the dehydrogenase activity of C6 cells was reduced to approximately 75% under two-hour lactacidosis (Figure 9, B and C), but the reduction of the enzyme activity was alleviated to approximately 95% by the VacA pretreatment (Figure 9, E and F). Thus, it was showed that the VacA pretreatment exhibited significant inhibitory effect on the glial cell death.

To examine whether the effects given by the VacA are dose-dependent, VacA pretreatments with three different concentrations were tested. Results showed that, as seen in D to F of Figure 8 and D to F of Figure 9, the effects given by the VacA were dose-dependent both in one-hour lactacidosis treatment and two-hour lactacidosis treatment: a higher VacA concentration had a higher cell death inhibitory effect. Specifically, the VacA concentration of 2.5 µg/ml or more is preferable.

These results showed that the introduction of anion channel by the VacA made it possible to attain RVD from cell swelling under lactacidosis and thereby rescued necrotic death of glial cells under lactacidosis.

### (3) Conclusion

It is concluded that under lactacidosis conditions, glial cell swelling was triggered by uptake of protons and lactic acid via MCT (Monocarboxylic Acid Transporter), and became sustained without volume regulation. The loss of volume regulation resulted from the impairment of volume-sensitive Cl⁻ channel activity. The volume regulation following lactacidosis-induced swelling was attained by the introduction of exogenous anion channels formed by VacA, which is an anion ionophore, but not by cation channels formed by gramicidin, which is a cation ionophore.

Lactacidosis occurring in cerebral ischemia is a major cause of cytotoxic brain edema. Glial cells are most likely a predominant cell type involved in cytotoxic edema. Thus, the inhibition of lactacidesis-induced glial cell swelling so as to reduce the cell volume (i.e. to cause RVD) is very effective in developing protective and therapeutic methods for brain edema caused by cerebral ischemia.

The present study demonstrated that inhibition of a volume-sensitive anion channel contributes to persistent swelling induced by lactacidosis in glial cells and that the introduction of VacA-mediated anion conductance to glial cells restored the ability to regulate volume after transient lactacidosis-induced swelling.

As described above, it was proved that the cell death of glial cells under lactacidosis can be inhibited by administrating, to the glial cells, the VacA protein or glycine receptor channel variant peptide (the VacA protein and glycine receptor channel variant peptide are anion ionophores). It is concluded that this cell death inhibitory mechanism is attained by the restoration of the cell volume from the persistent cell swelling. This mechanism is totally novel and distinctive from any ever-developed drugs and methods for treating ischemic disease. Moreover, it is known that not only glial cells but also neurons are also persistently swelled by the same mechanism under lactacidosis. It is expected that there is a high possibility that the cell death inhibitory method according to the present invention is applicable for inhibiting neuronal death, which causes neuronal degenerative disease.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

As described above, a method according to the present invention for inhibiting cell death is so arranged as to include administrating anion channel-forming peptide to cells under lactacidosis. A cell death inhibitor according to the present invention contains anion channel-forming peptide.

The method and inhibitor according to present invention can inhibit the cell swelling occurred under lactacidosis in ischemia, and subsequent cell death (especially, necrotic cell death). Therefore, the method and inhibitor according to the present invention are applicable for preventive and therapeutic use for various disease caused by the cell death. Furthermore, the method and inhibitor according to the present invention are highly useful because they use a new mechanism totally different from one used in conventional drugs for ischemic impairment.

A drug according to the present invention for treating contains the cell death inhibitor and is used for treating various disease caused by cell death. The drug can be used as a medicine for inhibiting, not only in brains but also in hearts and the like organs, ischemic cell death and organopathy associated with various disease. The drug according to the present invention is expected to make a large contribution to better life prognosis and various ischemic disease and be highly useful.

## Claims

1. A method for inhibiting cell death, comprising:
administrating an anion channel-forming peptide to cells under lactacidosis.

2. A method as set forth in Claim 1, wherein the cell death is necrotic cell death.

3. A method as set forth in Claim 1 or 2, wherein the cell death is judged based on nuclear stainability by cell membrane impermeable fluorescent dye propidium iodine.

4. A method as set forth in Claim 1 or 2, wherein the cell death is accompanied with significant reduction in mitochondrial dehydrogenase activity.

5. A method as set forth in any one of Claims 1 to 4, wherein the anion channel-forming peptide is a VacA protein derived from *Helicobacter pylori.*

6. A method as set forth in any one of Claims 1 to 4, wherein the anion channel-forming peptide is a glycine receptor channel variant peptide.

7. A cell death inhibitor comprising an anion channel-forming peptide.

8. A cell death inhibitor as set forth in Claim 7 inhibiting necrotic cell death.

9. A cell death inhibitor as set forth in Claim 7 or 8, wherein inhibition of the necrotic cell death is evaluated according to loss of nuclear stainability by cell membrane impermeable fluorescent dye propidium iodide.

10. A cell death inhibitor as set forth in Claim 7 or 8, wherein inhibition of the necrotic cell death is evaluated by prevention of reduction of mitochondrial dehydrogenase activity.

11. A cell death inhibitor as set forth in any one of Claims 7 to 10, wherein the anion channel-forming peptide is a VacA protein derived from *Helicobacter pylori*.

12. A cell death inhibitor as set forth in any one of Claims 7 to 10, wherein the anion channel-forming peptide is a glycine receptor channel variant peptide.

13. A therapeutic drug comprising a cell death inhibitor as set forth in any one of Claims 7 to 12 and being used for treating disease caused by cell death.

14. A therapeutic drug as set forth in Claim 13, used for treating disease caused by glial cell death.
